# FASCICULE DE BREVET EUROPEEN

(11) **EP 0 974 337 B1**
(45) Date de publication et mention de la délivrance du brevet: **21.03.2001**
(21) Numéro de dépôt: 99401643.4
(22) Date de dépôt: 01.07.1999
(51) Int. Cl.: A61K 7/13

(54) **Compositions pour la teinture des fibres kératiniques contenant des dérivés de 3-aminopyrazolines à titre de coupleur, procédé de teinture et kit de tenture**
Färbemittel für keratinische Faser enthaltend 3-Amino-Pyrazolinderivate als Kuppler, Verfahren zum Färben und Kit zum Färben
Keratinous fibre dyeing compositions containing 3-amino pyrazoline derivatives as coupling agents, process for dyeing and dyeing kit

(30) Priorité: 20.07.1998 FR 9809224
(43) Date de publication de la demande: 26.01.2000
(73) Titulaire: L'OREAL, 75008 Paris (FR)
(72) Inventeur: Breton, Philippe, 78150 Le Chesnay (FR); Lagrange, Alain, 77700 Coupvray (FR); Maubru, Mireille, 78400 Chatou (FR)
(74) Mandataire: Goulard, Sophie

(56) Documents cités:
- FR-A- 2 018 056
- FR-A- 2 355 834

## Description

L'invention a pour objet de nouvelles compositions pour la teinture d'oxydation des fibres kératiniques comprenant au moins une base d'oxydation et au moins un dérivé de 3-amino pyrazoline convenablement sélectionné à titre de coupleur, le procédé de teinture mettant en oeuvre cette composition et un agent oxydant, ainsi que le kit de teinture correspondant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet FR-A-2 018 056, d'utiliser certains dérivés de 3-amino pyrazolines à titre de bases d'oxydation, à savoir plus précisément des 1-(4'-aminophényl) 3-amino pyrazolines ou des 1-(4'-hydroxyphényl) 3-amino pyrazolines, pour la teinture d'oxydation des fibres kératiniques.

Or, la demanderesse vient maintenant de découvrir, de façon totalement inattendue et surprenante, que l'utilisation de certains dérivés de 3-amino pyrazoline de formule (I) définie ci-après, non seulement convient pour une utilisation comme coupleurs pour la coloration d'oxydation, mais en outre qu'ils conduisent à des colorations particulièrement puissantes notamment lorsqu'ils sont associés à des bases d'oxydation hétérocycliques. Ils permettent de plus d'obtenir des compositions tinctoriales conduisant à des colorations résistant bien aux diverses agressions que peuvent subir les cheveux. Enfin, ces composés s'avèrent être aisément synthétisables.

Ces découvertes sont à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation, et
- au moins un coupleur choisi parmi les dérivés de 3-amino pyrazoline de formule (I) suivante, et leurs sels d'addition avec un acide : 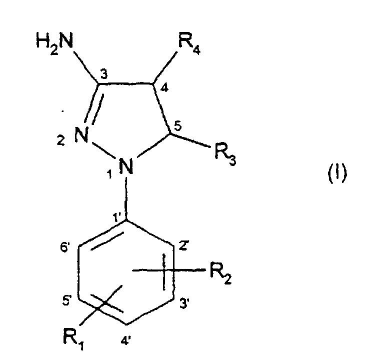
dans laquelle :
- R₁ et R₂, identiques ou différents, représentent un atome d'hydrogène ; un atome d'halogène ; un radical alkyle en C₁-C₄ un radical monohydroxyalkyle en C₁-C₄ un radical polyhydroxyalkyle en C₁-C₄ ; un radical alcoxy(C₁-C₄)alkyle en C₁-C₄ ; un-radical OR₅ dans lequel R₅ désigne un atome d'hydrogène, un radical alkyle en C₁-C₄, un radical aryle, un radical monohydroxyalkyle en C₁-C₄, un radical polyhydroxyalkyle en C₂-C₄ ou un radical alcoxy(C₁-C₄)alkyle en C₁-C₄
- R₃ et R₄, identiques ou différents, représentent un atome d'hydrogène ou d'halogène ; un radical alkyle en C₁-C₄ un radical monohydroxyalkyle en C₁-C₄ ; un radical polyhydroxyalkyle en C₂-C₄ ; un radical alcoxy(C₁-C₄)alkyle en C₁-C₄ ; un radical aminoalkyle en C₁-C₄ ; un radical aminoalkyle en C₁-C₄ dont l'amine est protégée par un radical acétyle, uréido ou sulfonyle ; un radical alkyl(C₁-C₄)aminoalkyle en C₁-C₄ ; un radical di-[(C₁-C₄)alkyl]aminoalkyle en C₁-C₄ (les dialkyles pouvant former un cycle aliphatique ou hétérocyclique à 5 ou 6 chaînons) ; un radical mono-[hydroxyalkyl(C₁-C₄)]-aminoalkyle en C₁-C₄ ou di-[hydroxyalkyl(C₁-C₄)]-aminoalkyle en C₁-C₄.

Comme indiqué précédemment, les colorations obtenues avec la composition de teinture d'oxydation conforme à l'invention sont puissantes, tout particulièrement lorsque les composés de formule (I) sont utilisés en association avec au moins une base d'oxydation hétérocyclique. Les compositions de teinture d'oxydation conformes à l'invention permettent de plus d'atteindre des nuances dans une très large palette de couleurs, présentant en outre d'excellentes propriétés de résistance vis à vis de l'action des différents agents extérieurs (lumière, intempéries, lavage, ondulation permanente, transpiration, frottements).

Dans la formule (I) ci-dessus, les atomes d'halogène sont choisis par le brome, le chlore, l'iode et le fluor et les radicaux alkyle et alcoxy peuvent être linéaires ou ramifiés.

Parmi les dérivés de 3-amino pyrazoline de formule (I) ci-dessus, utilisables à titre de coupleur dans les compositions tinctoriales conformes à l'invention, on peut notamment citer :
- la 3-amino 1-phényl pyrazoline,
- la 3-amino 1-phényl 4-méthyl pyrazoline,
- la 3-amino 1-phényl 5-méthyl pyrazoline,
- la 3-amino 1-phényl 5-éthyl pyrazoline,
- la 3-amino 1-phényl 5-n-propyl pyrazoline,
- la 3-amino 1-phényl 5-isopropyl pyrazoline,
- la 3-amino 1-(2'-bromo-phényl) pyrazoline,
- la 3-amino 1-(3'-bromo-phényl) pyrazoline,
- la 3-amino 1-(4'-bromo-phényl) pyrazoline,
- la 3-amino 1-(2'-chloro-phényl) pyrazoline,
- la 3-amino 1-(2',4'-dichloro-phényl) pyrazoline,
- la 3-amino 5-méthyl 1-(2',4'-dichloro-phényl) pyrazoline,
- la 3-amino 1-(3'-chloro-phényl) pyrazoline,
- la 3-amino 1-(2',5'-dichloro-phényl) pyrazoline,
- la 3-amino 1-(2',6'-dichloro-phényl) pyrazoline,
- la 3-amino 1-(3',4'-dichloro-phényl) pyrazoline,
- la 3-amino 1-(3',4'-dichloro-phényl) 4-méthyl pyrazoline,
- la 3-amino 1-(3',4'-dichloro-phényl)5-méthyl pyrazoline,
- la 3-amino 1-(3',4'-dichloro-phényl) 5-éthyl pyrazoline,
- la 3-amino 1-(3',5'-dichloro-phényl) pyrazoline,
- la 3-amino 1-(3'-chloro-4'-méthyl-phényl) pyrazoline,
- la 3-amino 5-méthyl 1-(3'-chloro-4'-méthyl-phényl ) pyrazoline,
- la 3-amino 1-(4'-amino-3'-chloro-phényl )pyrazoline,
- la 3-amino 1-(4'-chloro-phényl) pyrazoline,
- la 3-amino 1-(4'-chloro-phényl) 4-méthyl pyrazoline,
- la 3-amino 1-(4'-chloro-phényl) 5-méthyl pyrazoline,
- la 3-amino 1-(4'-chloro-phényl) 5-éthyl pyrazoline,
- la 3-amino 1-(4'-chloro-phényl) 5-n-propyl pyrazoline,
- la 3-amino 1-(4'-chloro-phényl) 4-n-butyl pyrazoline,
- la 3-amino 1-(4'-chloro-phényl) 5-n-butyl pyrazoline,
- la 3-amino 1-(2'-fluoro-phényl) pyrazoline,
- la 3-amino 1-(3'-fluoro-phényl) pyrazoline,
- la 3-amino 1-(4'-fluoro-phényl) pyrazoline,
- la 3-amino 1-(4'-fluoro-phényl) 5-fluoro pyrazoline,
- la 3-amino 1-(4'-fluoro-phényl) 4-méthyl pyrazoline,
- la 3-amino 1-(4'-fluoro-phényl) 5-méthyl pyrazoline,
- la 3-amino 1-(4'-fluoro-phényl) 5-éthyl pyrazoline,
- la 3-amino 1-(4'-fluoro-phényl) 5-n-butyl pyrazoline,
- la 3-amino 5-méthyl 1-(4'-fluoro-phényl) pyrazoline,
- la 3-amino 5-éthyl 1-(4'-fluoro-phényl) pyrazoline,
- la 3-amino 1-(4'-iodo-phényl) pyrazoline,
- la 3-amino 1-(2'-méthyl-phényl) pyrazoline,
- la 3-amino 1-(3'-méthyl-phényl) pyrazoline,
- la 3-amino 1-(4'-méthyl-phényl) pyrazoline,
- la 3-amino 1-(4'-t-butyl-phényl) pyrazoline,
- la 3-amino 1-(2'-méthoxy-phényl) pyrazoline,
- la 3-amino 1-(3'-méthoxy-phényl) pyrazoline,
- la 3-amino 1-(4'-méthoxy-phényl) pyrazoline,
- la 3-amino 1-(2'-éthoxy-phényl) pyrazoline,
- la 3-amino 1-(3'-éthoxy-phényl) pyrazoline,
- la 3-amino 1-(4'-éthoxy-phényl) pyrazoline,
- la 3-amino 1-(4',3'-diméthoxy-phényl) pyrazoline,
et leurs sels d'addition avec un acide.

Parmi ces dérivés de 3-amino pyrazoline de formule (I), on préfère plus particulièrement :
- la 3-amino 1-phényl pyrazoline,
- la 3-amino 1-(4'-fluoro-phényl) pyrazoline,
- la 3-amino 1-(4'-méthoxy-phényl) pyrazoline,
et leurs sels d'addition avec un acide.

Le ou les dérivés de 3-amino pyrazoline de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature des bases d'oxydation utilisées conformément à l'invention n'est pas critique. Elles peuvent être choisies parmi les bases d'oxydation classiquement utilisées en teinture d'oxydation et parmi lesquelles on peut notamment citer les paraphénylènediamines, les bases doubles, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bases doubles, on peut plus particulièrement citer à titre d'exemple, le N,N'-bis-(β-hydroxyéthyl)N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques et leurs sels d'addition avec un acide. Les compositions tinctoriales contenant l'association d'un coupleur de formule (I) et d'au moins une base d'oxydation hétérocycliques sont particulièrement préférées selon l'invention, car elles conduisent à des colorations particulièrement puissantes.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des coupleurs de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 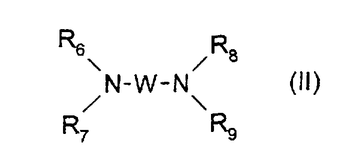 dans laquelle W est un reste propylène éventuellement substitué par un groupement hydroxyle ou un radical alkyle en C₁-C₆ ; R₆, R₇, R₈ et R₉, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est l'utilisation des dérivés de 3-amino pyrazoline de formule (I) ci-dessus à titre de coupleur pour la teinture d'oxydation des fibres kératiniques et en particulier des fibres kératiniques humaines telles que les cheveux.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases et les oxydo-réductases à 2 électrons. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les dérivés de 3-amino pyrazoline de formule (I) utilisés à titre de coupleur dans les compositions tinctoriales conformes à l'invention sont des composés connus qui peuvent être préparés par exemple selon un procédé en deux étapes connu dans la littérature et tel que décrit par exemple par F. Haviv et al., J. Med. Chem. **1988**, 31, 1719-1728 et J-L. Barascut et al., Bull. Soc. Chim. Fr. **1970**, 4, 1571-1576.

### EXEMPLES

### EXEMPLES 1 à 8 DE TEINTURE EN MILIEU ALCALIN

On a préparé les compositions tinctoriales, conformes à l'invention, suivantes (teneurs en grammes):

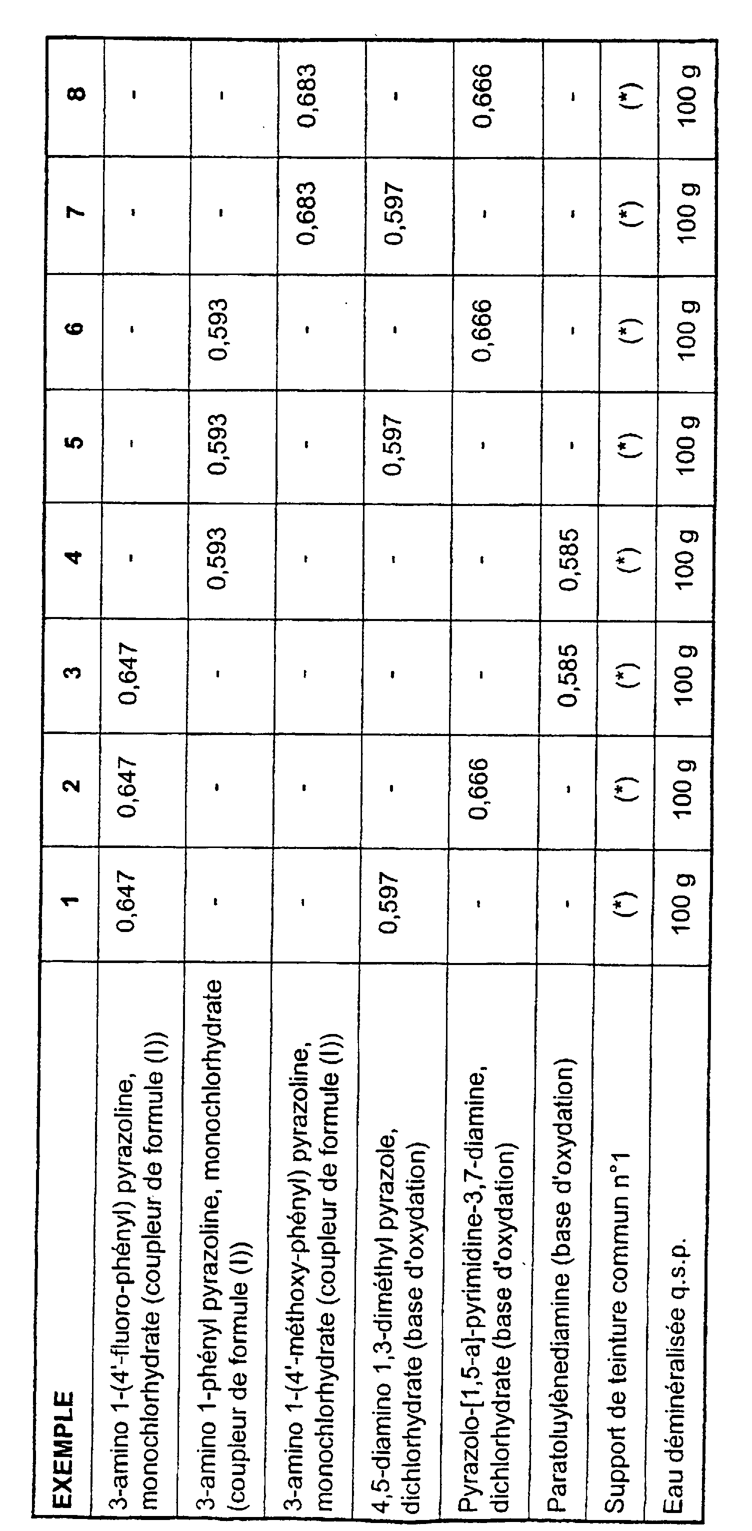

### (*) support de teinture commun n°1 :

- Alcool éthylique à 96° 18 g
- Métabisulfite de sodium en solution aqueuse à 35% 0,68 g
- Sel pentasodique de l'acide diéthylène triamino pentacétique 1,1 g
- Ammoniaque à 20% de NH₃ 10 g
- Eau déminéralisée q.s.p. 100 g

Au moment de l'emploi, on a mélangé chaque composition tinctoriale avec une quantité égale en poids d'une composition oxydante constituée par une solution de peroxyde d'hydrogène à 20 volumes (6 % en poids).

Chaque composition résultante présentait un pH de l'ordre de 10 ± 0,2, et a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** |
|---|---|
| **1** | Bleu légèrement cendré |
| **2** | Bleu puissant |
| **3** | Blond mat |
| **4** | Blond clair naturel |
| **5** | Blond clair cendré |
| **6** | Châtain clair bleuté |
| **7** | Blond très clair légèrement bleu vert |
| **8** | Bleu légèrement violacé |

### EXEMPLES 9 à 17 DE TEINTURE EN MILIEU NEUTRE

On a préparé les compositions tinctoriales, conformes à l'invention, suivantes (teneurs en grammes):

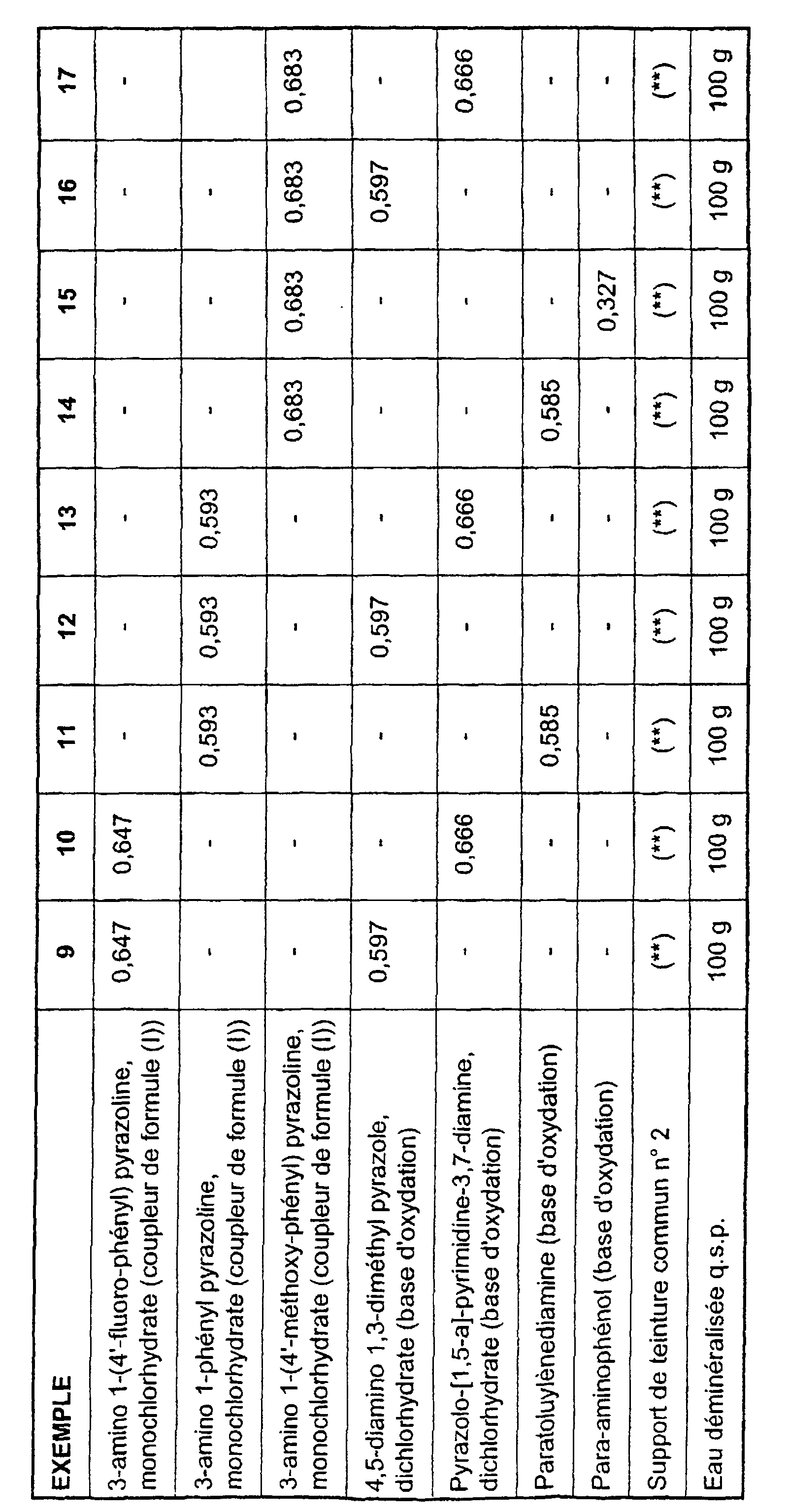

### (**) Support de teinture commun n°2 :

- Ethanol à 96° 18 g
- Métabisulfite de sodium en solution aqueuse à 35 % 0,68 g
- Sel pentasodique de l'acide diéthylènetriaminopentacétique 1,1 g
- Tampon K₂HPO₄ / KH₂PO₄ (1,5M / 1M) 10 g

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante présentait un pH de l'ordre de 6,8 ± 0,2, et a été appliquée pendant 30 minutes sur des mèches de cheveux gris naturels à 90 % de blancs. Les mèches de cheveux ont ensuite été rincées, lavées avec un shampooing standard puis séchées.

Les nuances obtenues figurent dans le tableau ci-dessous :

| **EXEMPLE** | **NUANCE SUR CHEVEUX NATURELS** |
|---|---|
| **9** | Turquoise |
| **10** | Bleu |
| **11** | Blond cendré irisé |
| **12** | Bleu légèrement vert |
| **13** | Bleu |
| **14** | Blond naturel cendré |
| **15** | Blond doré mat légèrement cuivré |
| **16** | Bleu vert |
| **17** | Bleu cendré |

## Revendications

1. Composition pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation, et
- au moins un coupleur choisi parmi les dérivés de 3-amino pyrazoline de formule (I) suivante, et leurs sels d'addition avec un acide : 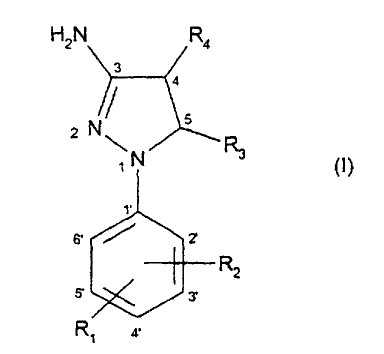
dans laquelle :
- R₁ et R₂, identiques ou différents, représentent un atome d'hydrogène ; un atome d'halogène ; un radical alkyle en C₁-C₄ ; un radical monohydroxyalkyle en C₁-C₄ ; un radical polyhydroxyalkyle en C₁-C₄ un radical alcoxy(C₁-C₄)alkyle en C₁-C₄ un radical OR₅ dans lequel R₅ désigne un atome d'hydrogène, un radical alkyle en C₁-C₄, un radical aryle, un radical monohydroxyalkyle en C₁-C₄, un radical polyhydroxyalkyle en C₂-C₄ ou un radical alcoxy(C₁-C₄)alkyle en C₁-C₄ ;
- R₃ et R₄, identiques ou différents, représentent un atome d'hydrogène ou d'halogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle en C₁-C₄ ; un radical aminoalkyle en C₁-C₄ ; un radical aminoalkyle en C₁-C₄ dont l'amine est protégée par un radical acétyle, uréido ou sulfonyle ; un radical alkyl(C₁-C₄)aminoalkyle en C₁-C₄ ; un radical di-[(C₁-C₄)alkyl]aminoalkyle en C₁-C₄ (les dialkyles pouvant former un cycle aliphatique ou hétérocyclique à 5 ou 6 chaînons) ; un radical mono-[hydroxyalkyl(C₁-C₄)]-aminoalkyle en C₁-C₄ ou di-[hydroxyalkyl(C₁-C₄)]-aminoalkyle en C₁-C₄.

2. Composition selon la revendication 1, caractérisée par le fait que les dérivés de 3-amino pyrazoline de formule (I) sont choisis parmi :
- la 3-amino 1-phényl pyrazoline,
- la 3-amino 1-phényl 4-méthyl pyrazoline,
- la 3-amino 1-phényl 5-méthyl pyrazoline,
- la 3-amino 1-phényl 5-éthyl pyrazoline,
- la 3-amino 1-phényl 5-n-propyl pyrazoline,
- la 3-amino 1-phényl 5-isopropyl pyrazoline,
- la 3-amino 1-(2'-bromo-phényl) pyrazoline,
- la 3-amino 1-(3'-bromo-phényl) pyrazoline,
- la 3-amino 1-(4'-bromo-phényl) pyrazoline,
- la 3-amino 1-(2'-chloro-phényl) pyrazoline,
- la 3-amino 1-(2',4'-dichloro-phényl) pyrazoline,
- la 3-amino 5-méthyl 1-(2',4'-dichloro-phényl) pyrazoline,
- la 3-amino 1-(3'-chloro-phényl) pyrazoline,
- la 3-amino 1-(2',5'-dichloro-phényl) pyrazoline,
- la 3-amino 1-(2',6'-dichloro-phényl) pyrazoline,
- la 3-amino 1-(3',4'-dichloro-phényl) pyrazoline,
- la 3-amino 1-(3',4'-dichloro-phényl) 4-méthyl pyrazoline,
- la 3-amino 1-(3',4'-dichloro-phényl) 5-méthyl pyrazoline,
- la 3-amino 1-(3',4'-dichloro-phényl) 5-éthyl pyrazoline,
- la 3-amino 1-(3',5'-dichloro-phényl) pyrazoline,
- la 3-amino 1-(3'-chloro-4'-méthyl-phényl) pyrazoline,
- la 3-amino 5-méthyl 1-(3'-chloro-4'-méthyl-phényl) pyrazoline,
- la 3-amino 1-(4'-amino-3'-chloro-phényl)pyrazoline,
- la 3-amino 1-(4'-chloro-phényl) pyrazoline,
- la 3-amino 1-(4'-chloro-phényl) 4-méthyl pyrazoline,
- la 3-amino 1-(4'-chloro-phényl) 5-méthyl pyrazoline,
- la 3-amino 1-(4'-chloro-phényl) 5-éthyl pyrazoline,
- la 3-amino 1-(4'-chloro-phényl) 5-n-propyl pyrazoline,
- la 3-amino 1-(4'-chloro-phényl) 4-n-butyl pyrazoline,
- la 3-amino 1-(4'-chloro-phényl) 5-n-butyl pyrazoline,
- la 3-amino 1-(2'-fluoro-phényl) pyrazoline,
- la 3-amino 1-(3'-fluoro-phényl) pyrazoline,
- la 3-amino 1-(4'-fluoro-phényl) pyrazoline,
- la 3-amino 1-(4'-fluoro-phényl) 5-fluoro pyrazoline,
- la 3-amino 1-(4'-fluoro-phényl)4-méthyl 4-méthyl pyrazoline,
- la 3-amino 1-(4'-fluoro-phényl) 5-méthyl pyrazoline,
- la 3-amino 1-(4'-fluoro-phényl) 5-éthyl pyrazoline,
- la 3-amino 1-(4'-fluoro-phényl) 5-n-butyl pyrazoline,
- la 3-amino 5-méthyl 1-(4'-fluoro-phényl) pyrazoline,
- la 3-amino 5-éthyl 1-(4'-fluoro-phényl) pyrazoline,
- la 3-amino 1-(4'-iodo-phényl) pyrazoline,
- la 3-amino 1-(2'-méthyl-phényl) pyrazoline,
- la 3-amino 1-(3'-méthyl-phényl) pyrazoline,
- la 3-amino 1-(4'-méthyl-phényl) pyrazoline,
- la 3-amino 1-(4'-t-butyl-phényl) pyrazoline,
- la 3-amino 1-(2'-méthoxy-phényl) pyrazoline,
- la 3-amino 1-(3'-méthoxy-phényl)pyrazoline,
- la 3-amino 1-(4'-méthoxy-phényl) pyrazoline,
- la 3-amino 1-(2'-éthoxy-phényl) pyrazoline,
- la 3-amino 1-(3'-éthoxy-phényl) pyrazoline,
- la 3-amino 1-(4'-éthoxy-phényl) pyrazoline,
- la 3-amino 1-(4',3'-diméthoxy-phényl) pyrazoline,
et leurs sels d'addition avec un acide.

3. Composition selon la revendication 2, caractérisée par le fait que les dérivés de 3-amino pyrazoline de formule (I), sont choisis parmi :
- la 3-amino 1-phényl pyrazoline,
- la 3-amino 1-(4'-fluoro-phényl) pyrazoline,
- la 3-amino 1-(4'-méthoxy-phényl) pyrazoline,
et leurs sels d'addition avec un acide

4. Composition selon l'une quelconque des revendications précédentes, caractérisée par le fait que le ou les dérivés de 3-amino pyrazoline de formule (I) et/ou le ou leurs sels d'addition avec un acide représentent de 0,0005 à 12 % en poids du poids total de la composition tinctoriale.

5. Composition selon la revendication 4, caractérisée par le fait que le ou les dérivés de 3-amino pyrazoline de formule (I) et/ou le ou leurs sels d'addition avec un acide représentent de 0,005 à 6 % en poids du poids total de la composition tinctoriale.

6. Composition selon l'une quelconque des revendications précédentes, caractérisée par le fait que les bases d'oxydation sont choisies parmi les paraphénylènediamines, les bis-phénylalkylènediamines, les para-aminophénols, les ortho-aminophénols et les bases hétérocycliques.

7. Composition selon la revendication 6, caractérisée par le fait que les paraphénylènediamines sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis(β-hydroxyéthyl) amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

8. Composition selon la revendication 6, caractérisée par le fait que les bases doubles sont choisies parmi le N,N'-bis(β-hydroxyéthyl)N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

9. Composition selon la revendication 6, caractérisée par le fait que les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

10. Composition selon la revendication 6, caractérisée par le fait que les ortho-aminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

11. Composition selon la revendication 6, caractérisée par le fait que les bases hétérocycliques sont choisies parmi les dérivés pyridiniques, les dérivés pyrimidiniques, les dérivés pyrazoliques, les dérivés pyrazolo-pyrimidiniques, et leurs sels d'addition avec un acide.

12. Composition selon l'une quelconque des revendications précédentes, caractérisée par le fait que la ou les bases d'oxydation représentent de 0,0005 à 12 % en poids du poids total de la composition tinctoriale.

13. Composition selon la revendication 12, caractérisée par le fait que la ou les bases d'oxydation représentent de 0,005 à 6 % en poids du poids total de la composition tinctoriale.

14. Composition selon l'une quelconque des revendications précédentes, caractérisée par le fait qu'elle renferme au moins un coupleur additionnel choisi parmi les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

15. Composition selon l'une quelconque des revendications précédentes, caractérisée par le fait que les sels d'addition avec un acide sont choisis parmi les chlorhydrates, les bromhydrates, les sulfates, les citrates, les succinates, les tartrates, les lactates et les acétates.

16. Composition selon l'une quelconque des revendications précédentes, caractérisée par le fait que le milieu approprié pour la teinture (ou support) est constitué par de l'eau ou par un mélange d'eau et d'au moins un solvant organique.

17. Utilisation des dérivés de 3-amino pyrazoline de formule (I) tels que définis à l'une quelconque des revendications 1 à 3 et 15, à titre de coupleur pour la teinture d'oxydation des fibres kératiniques et en particulier des fibres kératiniques humaines telles que les cheveux.

18. Procédé de teinture des fibres kératiniques et en particulier des fibres kératiniques humaines telles que les cheveux caractérisé par le fait que l'on applique sur ces fibres au moins une composition tinctoriale telle que définie à l'une quelconque des revendications 1 à 16, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

19. Procédé selon la revendication 18, caractérisé par le fait que l'agent oxydant présent dans la composition oxydante est choisi parmi le peroxyde d'hydrogène, le peroxyde d'urée, les bromates de métaux alcalins, les persels et les enzymes.

20. Dispositif à plusieurs compartiments, ou "kit" de teinture à plusieurs compartiments, dont un premier compartiment renferme une composition tinctoriale telle que définie à l'une quelconque des revendications 1 à 16 et un second compartiment renferme une composition oxydante.

## Patentansprüche

1. Zusammensetzung zum oxidativen Färben von Keratinfasern und insbesondere menschlichen Keratinfasern, wie dem Haar, dadurch gekennzeichnet, daß sie in einem zum Färben geeigneten Medium enthält:
- mindestens eine Oxidationsbase, und
- mindestens einen Kuppler, der unter den 3-Amino-pyrazolinderivaten der folgenden Formel (I) und deren Additionssalzen mit einer Säure ausgewählt ist: 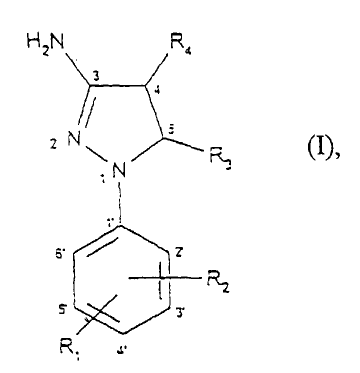 worin bedeuten:
- die Gruppen R₁ und R₂, die identisch oder voneinander verschieden sind, Wasserstoff; Halogen; C₁₋₄-Alkyl; C₁₋₄-Monohydroxyalk:yl; C₁₋₄-Polyhydroxyalkyl; C₁₋₄-Alkoxy-C₁₋₄-alkyl; eine Gruppe OR₅, wobei R₅ Wasserstoff, C₁₋₄Alkyl, Aryl, C₁₋₄-Monohydroxyalkyl, C₂₋₄-Polyhydroxyalkyl oder C₁₋₄-Alkoxy-C₁₋₄alkyl bedeutet;
- die Gruppen R₃ und R₄, die identisch oder voneinander verschieden sind, Wasserstoff; Halogen; C₁₋₄-Alkyl; C₁₋₄-Monohydroxyalkyl; C₂₋₄-Polyhydroxyalkyl; C₁₋₄-Alkoxy-C₁₋₄-alkyl; C₁₋₄-Aminoalkyl; C₁₋₄-Aminoalkyl, wobei die Aminogruppe mit einer Gruppe Acetyl, Ureido oder Sulfonyl geschützt ist; C₁₋₄-Alkyl-C₁₋₄₋aminoalkyl; Di-(C₁₋₄-alkyl)-C₁₋₄-aminoalkyl (wobei die beiden Alkylgruppen gemeinsam einen 5-oder 6-gliedrigen aliphatischen oder heterocyclischen Ring bilden können); Mono-(C₁₋₄-hydroxyalkyl)-C₁₋₄-aminoalkyl; oder Di-(C₁₋₄-hydroxyalkyl)-C₁₋₄-aminoalkyl.

2. Zusammensetzung nach Anspruch 1, dadurch gekennzeichnet, daß die 3-Aminopyrazolinderivate der Formel (I) ausgewählt sind unter: -3-Amino-1-phenyl-pyrazolin,
- 3-Amino-1-phenyl-4-methyl-pyrazolin,
- 3-Amino-1-phenyl-5-methyl-pyrazolin,
- 3-Amino-1-phenyl-5-ethyl-pyrazolin,
- 3-Amino-1-phenyl-5-n-propyl-pyrazolin,
- 3-Amino-1-phenyl-5-isopropyl-pyrazolin,
- 3-Amino-1-(2'-brom-phenyl)-pyrazolin,
- 3-Amino-1-(3'-brom-phenyl)-pyrazolin,
- 3-Amino-1-(4'-brom-phenyl)-pyrazolin,
- 3-Amino-1-(2'-chlor-phenyl)-pyrazolin,
- 3-Amino-1-(2',4'-dichlor-phenyl)-pyrazolin,
- 3-Amino-5-methyl-1-(2',4'-dichlor-phenyl)-pyrazolin,
- 3-Amino-1-(3'-chlor-phenyl)-pyrazolin,
- 3-Amino-1-(2',5'-dichlor-phenyl)-pyrazolin,
- 3-Amino-1-(2',6'-dichlor-phenyl)-pyrazolin,
- 3-Amino-1-(3',4'-dichlor-phenyl)-pyrazolin,
- 3-Amino-1-(3',4'-dichlor-phenyl)-4-methyl-pyrazolin,
- 3-Amino-1-(3',4'-dichlor-phenyl)-5-methyl-pyrazolin,
- 3-Amino-1-(3',4'-dichlor-phenyl)-5-ethyl-pyrazolin,
- 3-Amino-1-(3',5'-dichlor-phenyl)-pyrazolin,
- 3-Amino-1-(3'-chlor-4'-methyl-phenyl)-pyrazolin,
- 3-Amino-5-methyl-1-(3'-chlor-4'-methyl-phenyl)-pyrazolin,
- 3-Amino-1-(4'-amino-3'-chlor-phenyl)-pyrazolin,
- 3-Amino-1-(4'-chlor-phenyl)-pyrazolin,
- 3-Amino-1-(4'-chlor-phenyl)-4-methyl-pyrazolin,
- 3-Amino-1-(4'-chlor-phenyl)-5-methyl-pyrazolin,
- 3-Amino-1-(4'-chlor-phenyl)-5-ethyl-pyrazolin,
- 3-Amino-1-(4'-chlor-phenyl)-5-n-propyl-pyrazolin,
- 3-Amino-1-(4'-chlor-phenyl)-4-n-butyl-pyrazolin,
- 3-Amino-1-(4'-chlor-phenyl)-5-n-butyl-pyrazolin,
- 3-Amino-1-(2'-fluor-phenyl)-pyrazolin,
- 3-Amino-1-(3'-fluor-phenyl)-pyrazolin,
- 3-Amino-1-(4'-fluor-phenyl)-pyrazolin,
- 3-Amino-1-(4'-fluor-phenyl)-5-fluor-pyrazolin,
- 3-Amino-1-(4'-fluor-phenyl)-4-methyl-pyrazolin,
- 3-Amino-1-(4'-fluor-phenyl)-5-methyl-pyrazolin,
- 3-Amino-1-(4'-fluor-phenyl)-5-ethyl-pyrazolin,
- 3-Amino-1-(4'-fluor-phenyl)-5-n-butyl-pyrazolin,
- 3-Amino-5-methyl-1-(4'-fluor-phenyl)-pyrazolin,
- 3-Amino-5-ethyl-1-(4'-fluor-phenyl)-pyrazolin,
- 3-Amino-1-(4'-iod-phenyl)-pyrazolin,
- 3-Amino-1-(2'-methyl-phenyl)-pyrazolin,
- 3-Amino-1-(3'-methyl-phenyl)-pyrazolin,
- 3-Amino-1-(4'-methyl-phenyl)-pyrazolin,
- 3-Amino-1-(4'-tert.-butyl-phenyl)-pyrazolin,
- 3-Amino-1-(2'-methoxy-phenyl)-pyrazolin,
- 3-Amino-1-(3'-methoxy-phenyl)-pyrazolin,
- 3-Amino-1-(4'-methoxy-phenyl)-pyrazolin,
- 3-Amino-1-(2'-ethoxy-phenyl)-pyrazolin,
- 3-Amino-1-(3'-ethoxy-phenyl)-pyrazolin,
- 3-Amino-1-(4'-ethoxy-phenyl)-pyrazolin,
- 3-Amino-1-(4',3'-dimethoxy-phenyl)-pyrazolin, und
- deren Additionssalze mit einer Säure.

3. Zusammensetzung nach Anspruch 2, dadurch gekennzeichnet, daß die 3-Aminopyrazolinderivate der Formel (I) ausgewählt sind unter:
- 3-Amino-1-phenyl-pyrazolin,
- 3-Amino-1-(4'-fluor-phenyl)-pyrazolin,
- 3-Amino-1-(4'-methoxy-phenyl)-pyrazolin, und
- deren Additionssalze mit einer Säure.

4. Zusammensetzung nach einem der vorhergehenden Ansprüche, dadurch gekennzeichnet, daß das oder die 3-Aminopyrazolinderivate der Formel (I) und/oder deren Additionssalze mit einer Säure 0,0005 bis 12 Gew.-% des Gesamtgewichts der Färbemittelzusammensetzung ausmachen.

5. Zusammensetzung nach Anspruch 4, dadurch gekennzeichnet, daß das oder die 3-Aminopyrazolinderivate der Formel (I) und/oder deren Additionssalze mit einer Säure 0,005 bis 6 Gew.-% des Gesamtgewichts der Färbemittelzusammensetzung ausmachen.

6. Zusammensetzung nach einem der vorhergehenden Ansprüche, dadurch gekennzeichnet, daß die Oxidationsbase(n) unter den p-Phenylendiaminen, Bisphenylalkylendiaminen, p-Aminophenolen, o-Aminophenolen und heterocyclischen Basen ausgewählt sind.

7. Zusammensetzung nach Anspruch 6, dadurch gekennzeichnet, daß die p-Phenylendiamine unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-N,N-Bis(β-hydroxyethyl)amino-2-methyl-anilin, 4-N,N-Bis(β-hydroxyethyl)-amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3 -methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, N-β-Hydroxyethyloxy-p-phenylendiamin, 2-β-Acetylaminoethyloxy-p-phenylendiamin, N-(β-Methoxyethyl)-p-phenylendiamin und deren Additionssalze mit einer Säure ausgewählt sind.

8. Zusammensetzung nach Anspruch 6, dadurch gekennzeichnet, daß die Doppelbasen unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'-amino, 3'-methylphenyl)-ethylendiamin, 1,8-Bis(2,5-diaminophenoxy)-3,5-dioxaoctan und deren Additionssalze mit einer Säure ausgewählt sind.

9. Zusammensetzung nach Anspruch 6, dadurch gekennzeichnet, daß die p-Aminophenole unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalze mit einer Säure ausgewählt sind.

10. Zusammensetzung nach Anspruch 6, dadurch gekennzeichnet, daß die o-Aminophenole unter 2-Aminophenol, 2-Amino-5-methyl-phenol, 2-Amino-6-methyl-phenol, 5-Acetamido-2-amino-phenol und deren Additionssalze mit einer Säure ausgewählt sind.

11. Zusammensetzung nach Anspruch 6, dadurch gekennzeichnet, daß die heterocyclischen Basen unter den Pyridinderivaten, Pyrimidinderivaten, Pyrazolderivaten, Pyrazolo-pyrimidinderivaten und deren Additionssalze mit einer Säure ausgewählt sind.

12. Zusammensetzung nach einem der vorhergehenden Ansprüche, dadurch gekennzeichnet, daß die Oxidationsbase(n) 0,0005 bis 12 Gew.-% des Gesamtgewichts der Färbemittelzusammensetzung ausmachen.

13. Zusammensetzung nach Anspruch 12, dadurch gekennzeichnet, daß die Oxidations-base(n) 0,005 bis 6 Gew.-% des Gesamtgewichts der Färbemittelzusammensetzung ausmachen.

14. Zusammensetzung nach einem der vorhergehenden Ansprüche, dadurch gekennzeichnet, daß sie mindestens einen zusätzlichen Kuppler enthält, der unter den m-Phenylendiaminen, m-Aminophenolen, m-Dihydroxybenzolen und den heterocyclischen Kupplern, wie beispielsweise Indolderivaten, Indolinderivaten, Pyridinderivaten und Pyrazolonen, und deren Additionssalze mit einer Säure ausgewählt ist.

15. Zusammensetzung nach einem der vorhergehenden Ansprüche, dadurch gekennzeichnet, daß die Additionssalze mit einer Säure unter den Hydrochloriden, Hydrobromiden, Sulfaten, Citraten, Succinaten, Tartraten, Lactaten und Acetaten ausgewählt sind.

16. Zusammensetzung nach einem der vorhergehenden Ansprüche, dadurch gekennzeichnet, daß das zum Färben geeignete Medium (oder der Träger) aus Wasser oder einem Gemisch von Wasser und mindestens einem organischen Lösungsmittel besteht.

17. Verwendung von 3-Amino-pyrazolinderivaten der oben definierten Formel (I) nach einem der Ansprüche 1 bis 3 und 15 als Kuppler zum oxidativen Färben von Keratinfasern und insbesondere menschlichen Keratinfasern, wie dem Haar.

18. Verfahren zum Färben von Keratinfasern und insbesondere menschlichen Keratinfasern, wie dem Haar, dadurch gekennzeichnet, daß auf die Fasern eine Färbemittelzusammensetzung nach einem der Ansprüche 1 bis 16 aufgetragen wird und die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

19. Verfahren nach Anspruch 18, dadurch gekennzeichnet, daß das das Oxidationsmittel, das in der oxidierenden Zusammensetzung vorliegt, unter Wasserstoffperoxid, Harnstoffperoxid, Alkalimetallbromaten, Salzen von Persäuren und Enzymen ausgewählt ist.

20. Vorrichtung mit mehreren Abteilungen oder "Kit" zum Färben mit mehreren Abteilungen, wobei eine erste Abteilung eine Färbemittelzusammensetzung nach einem der Ansprüche 1 bis 16 und eine zweite Abteilung eine oxidierende Zusammensetzung enthält.

## Claims

1. Composition for the oxidation dyeing of keratin fibres, and in particular of human keratin fibres such as the hair, characterized in that it comprises, in a medium which is suitable for dyeing:
- at least one oxidation base, and
- at least one coupler chosen from 3-aminopyrazoline derivatives of formula (I) below, and the addition salts thereof with an acid: 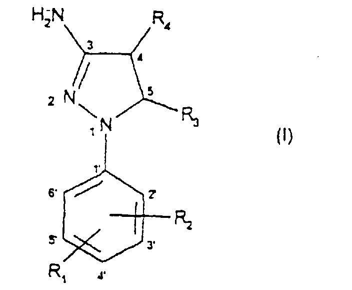 in which:
- R₁ and R₂, which may be identical or different, represent a hydrogen atom; a halogen atom; a C₁-C₄ alkyl radical; a C₁-C₄ monohydroxyalkyl radical; a C₁-C₄ polyhydroxyalkyl radical; a (C₁-C₄)alkoxy(C₁-C₄)alkyl radical; a radical OR₅ in which R₅ denotes a hydrogen atom, a C₁-C₄ alkyl radical, an aryl radical, a C₁-C₄ monohydroxyalkyl radical, a C₂-C₄ polyhydroxyalkyl radical or a (C₁-C₄) alkoxy (C₁-C₄) alkyl radical;
- R₃ and R₄, which may be identical or different, represent a hydrogen or halogen atom; a C₁-C₄ alkyl radical; a C₁-C₄ monohydroxyalkyl radical; a C₂-C₄ polyhydroxyalkyl radical; a (C₁-C₄)alkoxy(C₁-C₄)alkyl radical; a C₁-C₄ aminoalkyl radical; a C₁-C₄ aminoalkyl radical in which the amine is protected with an acetyl, ureido or sulphonyl radical; a (C₁-C₄)alkylamino(C₁-C₄)alkyl radical; a di[(C₁-C₄)alkyl]amino (C₁-C₄)alkyl radical (it being possible for the dialkyls to form a 5- or 6-membered aliphatic or heterocyclic ring); a mono [hydroxy(C₁-C₄) alkyl]amino(C₁-C₄)alkyl or di [hydroxy(C₁-C₄) alkyl] amino(C₁-C₄)alkyl radical.

2. Composition according to Claim 1, characterized in that the 3-aminopyrazoline derivatives of formula (I) are chosen from:
- 3-amino-1-phenylpyrazoline,
- 3-amino-1-phenyl-4-methylpyrazoline,
- 3-amino-1-phenyl-5-methylpyrazoline,
- 3-amino-1-phenyl-5-ethylpyrazoline,
- 3-amino-1-phenyl-5-n-propylpyrazoline,
- 3-amino-1-phenyl-5-isopropylpyrazoline,
- 3-amino-1-(2'-bromophenyl)pyrazoline,
- 3-amino-1-(3'-bromophenyl)pyrazoline,
- 3-amino-1-(4'-bromophenyl)pyrazoline,
- 3-amino-1-(2'-chlorophenyl)pyrazoline,
- 3-amino-1-(2', 4'-dichlorophenyl)pyrazoline,
- 3-amino-5-methyl-1-(2',4'-dichlorophenyl)-pyrazoline,
- 3-amino-1-(3'-chlorophenyl)pyrazoline,
- 3-amino-1-(2',5'-dichlorophenyl)pyrazoline,
- 3-amino-1-(2', 6'-dichlorophenyl)pyrazoline,
- 3-amino-1-(3',4'-dichlorophenyl)pyrazoline,
- 3-amino-1-(3',4'-dichlorophenyl)-4-methylpyrazoline,
- 3-amino-1-(3',4'-dichlorophenyl)-5-methylpyrazoline,
- 3-amino-1-(3',4'-dichlorophenyl)-5-ethylpyrazoline,
- 3-amino-1-(3',5'-dichlorophenyl)pyrazoline,
- 3-amino-1-(3'-chloro-4'-methylphenyl)pyrazoline,
- 3-amino-5-methyl-1-(3'-chloro-4'-methylphenyl)-pyrazoline,
- 3-amino-1-(4'-amino-3'-chlorophenyl)pyrazoline,
- 3-amino-1-(4'-chlorophenyl)pyrazoline,
- 3-amino-1-(4'-chlorophenyl)-4-methylpyrazoline,
- 3-amino-1-(4'-chlorophenyl)-5-methylpyrazoline,
- 3-amino-1-(4'-chlorophenyl)-5-ethylpyrazoline,
- 3-amino-1-(4'-chlorophenyl)-5-n-propylpyrazoline,
- 3-amino-1-(4'-chlorophenyl)-4-n-butylpyrazoline,
- 3-amino-1-(4'-chlorophenyl)-5-n-butylpyrazoline,
- 3-amino-1-(2'-fluorophenyl)pyrazoline,
- 3-amino-1-(3'-fluorophenyl)pyrazoline,
- 3-amino-1-(4'-fluorophenyl)pyrazoline,
- 3-amino-1-(4'-fluorophenyl)-5-fluoropyrazoline,
- 3-amino-1-(4'-fluorophenyl)-4-methylpyrazoline,
- 3-amino-1-(4'-fluorophenyl)-5-methylpyrazoline,
- 3-amino-1-(4'-fluorophenyl)-5-ethylpyrazoline,
- 3-amino-1-(4'-fluorophenyl)-5-n-butylpyrazoline,
- 3-amino-5-methyl-1-(4'-fluorophenyl)pyrazoline,
- 3-amino-5-ethyl-1-(4'-fluorophenyl)pyrazoline,
- 3-amino-1-(4'-iodophenyl)pyrazoline,
- 3-amino-1-(2'-methylphenyl)pyrazoline,
- 3-amino-1-(3'-methylphenyl)pyrazoline,
- 3-amino-1-(4'-methylphenyl)pyrazoline,
- 3-amino-1-(4'-tert-butylphenyl)pyrazoline,
- 3-amino-1-(2'-methoxyphenyl)pyrazoline,
- 3-amino-1-(3'-methoxyphenyl)pyrazoline,
- 3-amino-1-(4'-methoxyphenyl)pyrazoline,
- 3-amino-1-(2'-ethoxyphenyl)pyrazoline,
- 3-amino-1-(3'-ethoxyphenyl)pyrazoline,
- 3-amino-1-(4'-ethoxyphenyl)pyrazoline,
- 3-amino-1-(4',3'-dimethoxyphenyl)pyrazoline,
and the addition salts thereof with an acid.

3. Composition according to Claim 2,
characterized in that the 3-aminopyrazoline derivatives of formula (I) are chosen from:
- 3-amino-1-phenylpyrazoline,
- 3-amino-1-(4'-fluorophenyl)pyrazoline,
- 3-amino-1-(4'-methoxyphenyl)pyrazoline,
and the addition salts thereof with an acid.

4. Composition according to any one of the preceding claims, characterized in that the 3-aminopyrazoline derivative(s) of formula (I) and/or the addition salt(s) thereof with an acid represent(s) from 0.0005 to 12% by weight relative to the total weight of the dye composition.

5. Composition according to Claim 4, characterized in that the 3-aminopyrazoline derivative(s) of formula (I) and/or the addition salt(s) thereof with an acid represent(s) from 0.005 to 6% by weight relative to the total weight of the dye composition.

6. Composition according to any one of the preceding claims, characterized in that the oxidation bases are chosen from para-phenylenediamines, bis(phenyl)alkylenediamines, para-aminophenols, ortho-aminophenols and heterocyclic bases.

7. Composition according to Claim 6, characterized in that the para-phenylenediamines are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis (β-hydroxyethyl)-para-phenylenediamine, 4-amino-N,N-bis (β-hydroxyethyl)-2-methylaniline, 4-amino-2-chloro-N,N-bis (β-hydroxyethyl)aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N- (ethyl-β-hydroxyethyl)-para-phenylenediamine, N-(β, γ-dihydroxypropyl) -para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

8. Composition according to Claim 6, characterized in that the double bases are chosen from N,N'-bis(β-hydroxyethyl)-N,N'-bis(4'-aminophenyl)-1,3-diaminopropanol, N,N'-bis(β-hydroxyethyl)-N,N'-bis (4'-aminophenyl)ethylenediamine, N,N'-bis(4-aminophenyl)-tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis-(ethyl)-N,N'-bis (4'-amino-3'-methylphenyl)ethylenediamine and 1,8-bis(2,5-diaminophenoxy)-3,5-dioxaoctane, and the addition salts thereof with an acid.

9. Composition according to Claim 6, characterized in that the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

10. Composition according to Claim 6, characterized in that the ortho-aminophenols are chosen from 2-aminophenol, 2-amino-5-methylphenol, 2-amino-6-methylphenol and 5-acetamido-2-aminophenol, and the addition salts thereof with an acid.

11. Composition according to Claim 6, characterized in that the heterocyclic bases are chosen from pyridine derivatives, pyrimidine derivatives, pyrazole derivatives and pyrazolopyrimidine derivatives, and the addition salts thereof with an acid.

12. Composition according to any one of the preceding claims, characterized in that the oxidation base(s) represent(s) from 0.0005 to 12% by weight relative to the total weight of the dye composition.

13. Composition according to Claim 12, characterized in that the oxidation base(s) represent(s) from 0.005 to 6% by weight relative to the total weight of the dye composition.

14. Composition according to any one of the preceding claims, characterized in that it contains at least one additional coupler chosen from meta-phenylenediamines, meta-aminophenols, meta-diphenols and heterocyclic couplers such as, for example, indole derivatives, indoline derivatives, pyridine derivatives and pyrazolones, and the addition salts thereof with an acid.

15. Composition according to any one of the preceding claims, characterized in that the addition salts with an acid are chosen from the hydrochlorides, hydrobromides, sulphates, citrates, succinates, tartrates, lactates and acetates.

16. Composition according to any one of the preceding claims, characterized in that the medium which is suitable for dyeing (or support) consists of water or of a mixture of water and at least one organic solvent.

17. Use of the 3-aminopyrazoline derivatives of formula (I) as defined in any one of Claims 1 to 3 and 15, as couplers for the oxidation dyeing of keratin fibres, and in particular of human keratin fibres such as the hair.

18. Process for dyeing keratin fibres, and in particular human keratin fibres such as the hair,
characterized in that at least one dye composition as defined in any one of Claims 1 to 16 is applied to these fibres and in that the colour is developed at acidic, neutral or alkaline pH using an oxidizing agent which is added to the dye composition only at the time of use, or which is present in an oxidizing composition which is applied simultaneously or sequentially in a separate manner.

19. Process according to Claim 18, characterized in that the oxidizing agent present in the oxidizing composition is chosen from hydrogen peroxide, urea peroxide, alkali metal bromates, persalts and enzymes.

20. Multi-compartment device or multi-compartment dyeing "kit", a first compartment of which contains a dye composition as defined in any one of Claims 1 to 16 and a second compartment of which contains an oxidizing composition.
